# EUROPEAN PATENT SPECIFICATION

(11) **EP 3 970 616 B1**
(45) Date of publication and mention of the grant of the patent: **08.01.2025**
(21) Application number: 21197580.0
(22) Date of filing: 18.09.2021
(51) Int. Cl.: A61B 5/308, A61B 5/352, A61B 5/358, A61B 5/00

(54) **A DEVICE FOR DETECTING THE QRS COMPLEX OF ELECTROCARDIOGRAM SIGNAL**
VORRICHTUNG ZUR DETEKTION DES QRS-KOMPLEXES EINES ELEKTROKARDIOGRAMMSIGNALS
DISPOSITIF POUR DÉTECTER LE COMPLEXE QRS D'UN SIGNAL D'ÉLECTROCARDIOGRAMME

(30) Priority: 22.09.2020 PL 43539620
(43) Date of publication of application: 23.03.2022
(73) Proprietor: Akademia Gorniczo-Hutnicza im. Stanislawa Staszica w Krakowie, 30-059 Krakow (PL)
(72) Inventor: REKLEWSKI, Wojciech, 02-493 Warszawa (PL); HERYAN, Katarzyna, 30-619 Krakow (PL); AUGUSTYNIAK, Piotr, 31-457 Krakow (PL); MISKOWICZ, Marek, 31-621 Krakow (PL)
(74) Representative: Belz, Anna

(56) References cited:
- KR-A- 20160 107 390
- CHEN H C ET AL: "A moving average based filtering system with its application to real-time QRS detection", COMPUTERS IN CARDIOLOGY 2003. THESSALONIKI, GREECE, SEPT. 21 - 24, 2003; [COMPUTERS IN CARDIOLOGY], NEW YORK, NY : IEEE, US, vol. VOL. 30, 21 September 2003 (2003-09-21), pages 585 - 588, XP010698972, ISBN: 978-0-7803-8170-4, DOI: 10.1109/CIC.2003.1291223
- JALALEDDINE S ET AL: "AMBULATORY ECG WAVE DETECTION FOR AUTOMATED ANALYSIS: A REVIEW", ISA TRANSACTIONS, INSTRUMENT SOCIETY OF AMERICA. PITTSBURGH, US, vol. 26, no. 4, 1 January 1987 (1987-01-01), pages 33 - 43, XP000046086, ISSN: 0019-0578
- RAHUL JAGDEEP ET AL: "Dynamic thresholding based efficient QRS complex detection with low computational overhead", BIOMEDICAL SIGNAL PROCESSING AND CONTROL, vol. 67, 1 May 2021 (2021-05-01), NL, pages 102519, XP055885753, ISSN: 1746-8094, DOI: 10.1016/j.bspc.2021.102519

## Description

The subject of the invention is a device for detecting the QRS complex of an electrocardiogram (ECG) signal applicable in biomedical diagnostics.

A device for monitoring the ECG signal, consisting of technical means for measuring the pulse, filters and amplifiers for noise elimination and amplification of the diagnostically useful signal, and a computer programmed to suppress frequency components below 15 Hz and above 25 Hz and to amplify the R waves in the QRS signal is known from the American patent description US5738104.

There is a device and a method known from the patent US5188116 for electrocardiographic tests, the device which is a system for analyzing electrocardiographic activity for the detection of ischemic heart disease, consisting of tools for detecting a multiplicity of periodic electrocardiographic signals, a memory unit storing these signals, a microprocessor capable of calculating collective cycles for each signal, detecting and storing the amplitude characteristics of the signals, as well as determining the variance of the signals and modifying them, furthermore of technical means for determining the degree of coronary artery disease. The patent also describes a method of analyzing electrocardiographic activity for the detection of ischemic heart disease, including collecting and storing electrocardiographic signals, establishing a cumulative cycle for each signal, determining the variance of each signal and the total variability of all signals, and then determining the size of the coronary heart disease on their basis.

A method known from the article "A moving average based filtering system with its application to real-time QRS detection", Computers in Cardiology, 2003, pp. 585-588, by Chen, H. C., and Chen, S. W. comprises the calculation of a moving average of an input ECG signal, which its further subtracted from the input ECG signal. Next, the resultant signal is squared, and the moving average of the squared signal is calculated, which is an input to a decision block. The input signal to the decision block is compared to the threshold calculated as THRESHOLD(n) = alfa * gamma * PEAK + (1-alfa)THRESHOLD(n-1), while PEAK is a new local max in the input to the decision block, alfa and gamma are coefficients between 0 and 1, and n-1 is the index of the previous QRS detection cycle. The QRS is detected only when the peak level of the feature signal exceeds the threshold.

A device known from the patent application KR 2016 0107390 comprises an ECG signal detection module wherein an ECG signal is subject to a low pass filter and a high pass filter, as well as a control unit that differentiates an output signal of the pass filter. The controller sets a window of 80ms and averages an absolute value of the differentiated signal in a section within the set window to repeatedly obtain a peak value at a predetermined period of time.

The essence of a device for detecting the QRS complex in an electrocardiogram signal connected to a ECG signal measurement module is that it has an ABS_DIFF_SHORT_MODULE module that determines the signal being the difference of the current momentary value of the ECG signal and an average SHORT_AVG value of the ECG signal, calculated for a T_SHORT time segment of a fixed length, and an ABS_DIFF_LONG_MODULE module, which determines the signal being the difference of the current momentary value of the ECG signal and the average LONG_AVG value of the ECG signal calculated for the T_LONG time segment of a fixed length, the inputs of which are connected to the output of a ECG_MODULE measuring module of the ECG signal. The ABS_DIFF_SHORT_MODULE module output is connected to one of a COMP comparator inputs. The output of the ABS_DIFF_LONG_MODULE module is connected to the input of a PEAK_DETECTOR detection module, adapted to detect during the SEARCHING_WINDOW time window to search for the R wave in the QRS complex of the maximum value of the signal being the difference of the current instantaneous value of the ECG signal and its mean LONG_AVG value, calculated for the time segment T_LONG, is considered the amplitude of the R wave in the QRS complex, and also adapted to detect the instant of occurrence of the maximum value of the signal determined in this way, which is considered to be the instant of the R wave in the QRS complex. The output of the COMP comparator is connected to the input of the PULSE GENERATOR, which generates a pulse with the duration of the SEARCHING_WINDOW time window used to search for the R wave in the QRS complex of the ECG signal. The output of the PULSE GENERATOR pulse generator is connected to the gating input of the PEAK_DETECTOR detection module and to a R_AMPLITUDE_MEMORY memory recording module input to store information about the R wave amplitude of the ECG signal, and to a R_TIMESTAMP_MEMORY memory recording input to store information about the R-wave occurrence of the ECG signal. The PEAK_DETECTOR detection module output is also connected to the R_AMPLITUDE_MEMORY and R_TIMESTAMP_MEMORY memory module inputs. The output of the memory module R_AMPLITUDE_MEMORY is connected here with the input of a TH_MODULE module, adapted to determine the current threshold value TH on the basis of the amplitudes of R waves detected with the PEAK_DETECTOR detection module and stored with the memory module R_AMPLITUDE_MEMORY in the previous QRS complexes of the ECG signal. The TH_MODULE output is also connected to one of the COMP comparator inputs.

The device for detecting the QRS signal in the electrocardiogram signal, thanks to the subtraction operation of the current instantaneous value of the ECG signal to its average values, calculated respectively for the T_SHORT and T_LONG time segments in the ABS_DIFF_SHORT_MODULE and ABS_DIFF_LONG_MODULE modules, is resistant to noise and disturbances occurring during the measurement of the signal, electrocardiograms. In addition, by continuously adjusting the TH threshold value used to determine the start of the SEARCHING_WINDOW time window for searching for the R wave in the QRS complex to the TH threshold determined in the previous QRS detection cycle and to the amplitude of the R wave in the current QRS detection cycle, by means of the TH_MODULE module reduces the sensitivity of R-wave detection to transient fluctuations in the maximum range of changes in the value of the electrocardiogram signal that may occur during the measurement of the ECG signal.

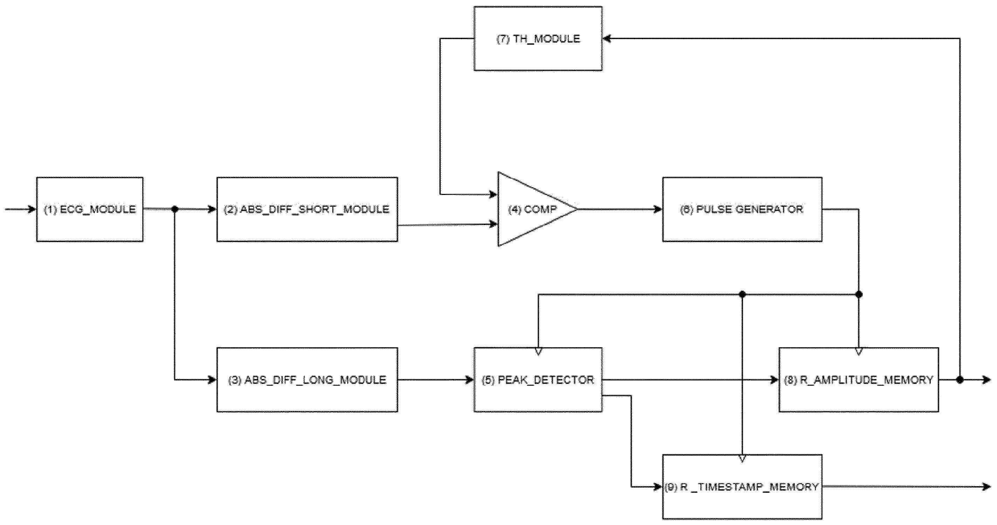

The subject matter of the invention is now shown in the drawing, which shows a block diagram of the device for detecting the QRS complex showing its modules and the connections between them.

List of device elements with a description of their functions:
(1) ECG_MODULE - electrocardiogram (ECG) signal measurement module,
(2) ABS_DIFF_SHORT_MODULE - module that determines the ABS_DIFF_SHORT signal, which is the difference between the current instantaneous value of the ECG signal and the average SHORT_AVG value of the ECG signal calculated for the fixed length T_SHORT time interval,
(3) a module that determines the ABS_DIFF_LONG signal, being the difference between the current instantaneous value of the ECG signal and the average LONG_AVG value of the ECG signal calculated for the T_LONG time interval of a predetermined length,
(4) COMP - comparator,
(5) PEAK_DETECTOR - detector of the maximum value of the ABS_DIFF_LONG signal during the SEARCHING_WINDOW time window for searching for the R wave in the QRS complex,
(6) PULSE GENERATOR - a pulse generator which, after the triggering signal appears, generates a pulse of a fixed duration equal to the SEARCHING_WINDOW time window for searching for the R wave in the QRS complex of the ECG signal,
(7) TH_MODULE - module that determines the threshold TH value for the ABS_DIFF_SHORT signal, used to define the beginning of the SEARCHING_WINDOW time window for searching for the R wave in the QRS complex of the ECG signal,
(8) R_AMPLITUDE_MEMORY - memory module for storing information on the amplitude of the maximum values of the LONG_ABS_DIFF signal in subsequent cycles of the ECG signal, which are considered as the amplitudes of R waves in subsequent QRS complexes of the ECG signal,
(9) R _TIMESTAMP_MEMORY - memory module for storing information about the times of the maximum LONG_ABS_DIFF signal values in subsequent cycles of the ECG signal, which are considered to be the times of R wave occurrence in subsequent QRS complexes of the ECG signal.

An ECG_MODULE 1 measurement module of the ECG signal includes a sampling-memory circuit that provides ECG signal samples at 250 Hz frequency and an analog-to-digital converter that converts the ECG signal sample values into a digital word with 6-bit resolution. An ABS_DIFF_SHORT_MODULE 2 and ABS_DIFF_LONG_MODULE 3 modules contain a microprocessor that calculates the difference between the digital representation of the current instantaneous value of the ECG signal and the corresponding mean SHORT_AVG value of the ECG signal, calculated for the T_SHORT time segment with a length of 55 ms and the average value of the LONG_AVG of the ECG signal for the T_LONG time segment with a length of 277 ms.

The PULSE GENERATOR 6 pulse generator is designed as a monostable trigger which, when triggered, generates a positive pulse with a duration of 200 ms. The pulse generated by the PULSE GENERATOR 6 pulse generator also determines the duration of the SEARCHING_WINDOW time window, during which the R wave is searched for in the QRS complex of the ECG signal and activates the PEAK_DETECTOR 5 detection module by connecting the PULSE GENERATOR 6 pulse generator output to the module's gating input (enable) of the PEAK_DETECTOR 5. The PEAK_DETECTOR 5 detection module comprises a microprocessor which, based on the output of the ABS_DIFF_LONG_MODULE 3 module, performs a detection of the maximum value of the ABS_DIFF_LONG signal during the SEARCHING_WINDOW time window. The detected maximum value of the ABS_DIFF_LONG signal during the SEARCHING_WINDOW time window is considered as information about the R wave amplitude. At the same time, the microprocessor in the PEAK_DETECTOR 5 detection module, using an internal clock signal generator, performs the determination of the intervals between the moments of occurrence of R waves in adjacent QRS of ECG signal complexes by calculating the number of clock signal periods within a time interval defined by the times of occurrence of R waves in adjacent QRS complexes of the ECG signal.

The output of the PULSE GENERATOR 6 pulse generator is connected to the input of the R_AMPLITUDE_MEMORY 8 recording module and to the output of R_TIMESTAMP_MEMORY 9. The trailing edge of the positive pulse generated by the pulse generator PULSE GENERATOR 6 writes to the R_AMPLITUDE_MEMORY 8 memory module information about the R wave amplitude and writes to the R_TIMESTAMP_MEMORY 9 memory module information about the times of occurrence of R waves in the QRS complexes of the ECG signal.

The TH_MODULE 7 module for determining the TH threshold value, used to detect the start of the timing using the PULSE GENERATOR 6 pulse generator, the beginning of the SEARCHING_WINDOW time window contains a microprocessor which, based on the TH threshold value determined by the TH_MODULE 7 module in the previous QRS detection cycle and stored in the R_AMPLITUDE_MEMORY 8 memory module of the R wave amplitude information in the QRS complex in the current ECG signal cycle determines a new TH threshold value to detect the start of the SEARCHING_WINDOW time window in the next ECG signal QRS detection cycle.

The electrocardiogram (ECG) signal, representing the electrical activity of the patient's heart, is received with the ECG_MODULE 1 measurement module via electrodes attached to the patient's body. The ECG signal can be reduced to a sequence of positive and negative deviations (waves) from the isoelectric line, which corresponds to the time periods during which no heart beats are detected. The group of the largest waves, called the QRS complex, consists of a negative deflection (Q wave), a positive deflection (R wave), and a negative second deflection (S wave). The R wave usually has the highest amplitude in the QRS complex. The detection of the QRS complex is often reduced to the detection of the R wave. The statistics of the time intervals between the R waves and the amplitudes of the R waves is an important diagnostic information, used in medicine, among other things, to recognize the work of the heart.

In the solution according to the invention, when detecting each QRS complex in the ECG signal by means of the ABS_DIFF_SHORT_MODULE 2 module, the ABS_DIFF_SHORT signal is determined on the basis of the ECG signal, which is the difference of the current instantaneous value of the ECG signal and the mean SHORT_AVG value of the ECG signal calculated for the T_SHORT time segment with a length equal to 55 ms. At the same time, the ABS_DIFF_LONG_MODULE 3 module determines the ABS_DIFF_LONG signal, which is the difference between the current instantaneous value of the ECG signal and the average LONG_AVG value of the ECG signal calculated for the T_LONG time interval of 277 ms. Monitoring of the ABS_DIFF_SHORT signal is aimed at detecting the beginning of the rising edge of the ECG signal preceding the occurrence of the R wave in the QRS complex, and subtracting the current value of the ECG signal from the average SHORT_AVG value is aimed at filtering out any noise and disturbances in the ECG signal received by the ECG_MODULE 1 measuring module.

The SHORT_ABS_DIFF signal available at the output of the ABS_DIFF_SHORT_MODULE 2 module is continuously compared by means of the COMP 4 comparator with the set value threshold TH, determined by the TH_MODULE 7 module. The detection of the ABS_DIFF_SHORT signal reaching a predetermined TH threshold is taken as the start of the rising edge of the ECG signal preceding the R-wave in the QRS complex. When the moment the ABS_DIFF_SHORT signal reaches the set TH value threshold is detected, the COMP 4 comparator, by means of an appropriate signal on its output, starts generating a 200 ms pulse using the PULSE GENERATOR 6 pulse generator, which determines the SEARCHING_WINDOW time window, during which the R wave is searched for in the QRS complex of the ECG signal. The pulse generated at the output of the pulse generator PULSE GENERATOR 6 with its active logic level activates the PEAK_DETECTOR 5 detection module, which records the maximum value of the ABS_DIFF_LONG signal generated at the output of the ABS_DIFF_LONG_MODULE 3 module during the SEARCHING_WINDOW time window.

Subtracting the current instantaneous value of the ECG signal from the average LONG_AVG value is designed to filter out possible noises and interferences in the ECG signal received by the ECG_MODULE 1 measuring module.

With the PEAK_DETECTOR 5 detection module, the instant of occurrence of the detected maximum value of the ABS_DIFF_LONG signal during the SEARCHING_WINDOW timezone is also recorded.

The maximum value of the ABS_DIFF_LONG signal produced at the output of the ABS_DIFF_LONG_MODULE 3 module during the SEARCHING_WINDOW time window is considered to be the R wave amplitude in the QRS complex of the ECG signal. In turn, the moment the R wave appears in the QRS complex of the ECG signal is considered to be the moment of the detected maximum value of the ABS_DIFF_LONG signal during the SEARCHING_WINDOW time window.

Then, when the trailing edge of the pulse generated at the output of the PULSE GENERATOR 6 pulse generator occurs, the maximum value of the LONG_ABS_DIFF signal previously detected by the PEAK_DETECTOR 5 module is recorded using the R_AMPLITUDE_MEMORY 8 module, and the moment of the maximum signal value is saved using the R_TIMESTAMP_MEMORY 9 module. ABS_DIFF_LONG during the SEARCHING_WINDOW time window.

Then, by means of the TH_MODULE 7 module, a new threshold value TH is determined for detecting the start of timing by the pulse generator PULSE GENERATOR 6 the beginning of the SEARCHING_WINDOW time window. The new threshold value TH is determined from the threshold value TH determined by the TH_MODULE 7 module in the previous QRS detection cycle and recorded with the PEAK_DETECTOR 5 detection module and the R-wave amplitude in the QRS complex stored in the R_AMPLITUDE_MEMORY 8 memory module in the current QRS complex detection cycle in such a way that the TH threshold in a given detection cycle of the QRS complex is the sum of the product of the TH threshold determined in the previous detection cycle of the QRS complex and a scaling factor less than one and the product of the R wave amplitude recorded in the current QRS complex detection cycle, one minus the scaling factor, and a weighting factor also less than one, where the threshold of the TH value before the first detection cycle of the QRS complex is determined using the TH_MODULE 7 module in the form of a product of a weighting factor lower than one and the maximum value of the ECG signal calculated for a time segment equal to one second. Then the value of the SHORT_ABS_DIFF signal produced at the output of the ABS_DIFF_SHORT_MODULE 2 module based on the ECG measurement signal provided by the ECG_MODULE 1 ECG measurement module is compared again with the set threshold of the TH value using the comparator COMP 4, and the cycle is repeated.

## Claims

1. A device for detecting a QRS complex of an electrocardiogram (ECG) signal with a connection to the ECG signal measurement module, wherein the device comprises an ABS_DIFF_SHORT_MODULE (2) module that determines the signal being the difference of the current instantaneous value of the ECG signal and an average SHORT_AVG value of the ECG signal calculated for a T_SHORT time interval with a predetermined length, and an ABS_DIFF_LONG_MODULE (3) module, which determines the signal being the difference of the current instantaneous value of the ECG signal and an average LONG_AVG value of the ECG signal calculated for the T_LONG time interval of a fixed length, inputs of which are connected to an output of a ECG_MODULE (1) measuring module of the ECG signal, while the ABS_DIFF_SHORT_MODULE (2) module output is connected to one of a COMP (4) comparator inputs, and the ABS_DIFF_LONG_MODULE (3) module output is connected to a PEAK_DETECTOR (3) detection module input, adapted to detect during a SEARCHING_WINDOW time window to search for a R wave in the QRS complex, the maximum value of the signal being the difference of the current instantaneous value of the ECG signal and its average LONG_AVG value calculated for the T_LONG time segment, which is considered the amplitude of the R wave in the QRS complex, and at the same time is adapted to detect the time of the occurrence of the maximum value of the signal determined in this way, which is considered to be the moment of the R wave occurrence in the QRS complex, with the output of COMP (4) comparator connected to the input of a PULSE GENERATOR (6) pulse generator, which generates the pulse with the time duration of the SEARCHING_WINDOW time window, which is used to search for the R wave in the QRS complex of the ECG signal, while the output of the PULSE GENERATOR (6) pulse generator is connected to the gating input of the PEAK_DETECTOR (5) detection module and to an input of R_AMPLITUDE_MEMORY (8) memory recording module for storing information about the R wave amplitude of the ECG signal, and also with the input of a R_TIMESTAMP_MEMORY (9) memory recording module for storing information about the R wave occurrence of the ECG signal, while the PEAK_DETECTOR (5) detection module output is also connected to the R_AMPLITUDE_MEMORY (8) and R_TIMESTAMP_MEMORY (9) memory module inputs, and the output of the memory module R_AMPLITUDE_MEMORY (8) is connected to the input of a TH_MODULE (7) module, adapted to determine a current threshold value TH based on the detected with the PEAK_DETECTOR (5) detection module and stored with the R_AMPLITUDE_MEMORY (8) amplitude of the R wave in the previous QRS complexes of the ECG signal, with the output of the TH_MODULE (7) module also connected to one of the comparator COMP (4) inputs.

## Patentansprüche

1. Vorrichtung zur Erkennung des QRS-Komplexes im Elektrokardiogrammsignal (EKG), die über eine Verbindung zum EKG-Signalmessmodul verfügt, wobei die Vorrichtung ein Modul ABS_DIFF_SHORT_MODULE (2) aufweist, das ein Signal aus der Differenz zwischen dem aktuellen Momentanwert des EKG-Signals und dem Durchschnittswert von SHORT_AVG des EKG-Signals, der über ein Zeitintervall T_SHORT mit festgelegter Länge berechnet wird, ermittelt, sowie ein Modul ABS_DIFF_LONG_MODULE (3) aufweist, das ein Signal aus der Differenz zwischen dem aktuellen Momentanwert des EKG-Signals und dem Durchschnittswert von LONG_AVG des EKG-Signals, der über ein Zeitintervall T_LONG mit festgelegter Länge berechnet wird, ermittelt, deren Eingänge mit dem Ausgang des Messmoduls ECG_MODULE (1) des EKG-Signals verbunden sind, wobei der Ausgang des Moduls ABS_DIFF_SHORT_MODULE (2) mit einem der Eingänge eines Komparators COMP (4) verbunden ist, und der Ausgang des Moduls ABS_DIFF_LONG_MODULE (3) mit einem Eingang des Erfassungsmoduls PEAK_DETECTOR (5) verbunden ist, das dazu geeignet ist, während des Zeitfensters SEARCHING_WINDOW die R-Zacke im QRS-Komplex zu erkennen, den Maximalwert des Signals aus der Differenz zwischen dem aktuellen Momentanwert des EKG-Signals und seinem Durchschnittswert von LONG_AVG des EKG, der über das Zeitintervall T_LONG berechnet wird, zu erfassen, der als Amplitude der R-Zacke im QRS-Komplex betrachtet wird, und gleichzeitig geeignet ist, den Zeitpunkt des Auftretens des auf diese Weise bestimmten Maximalwerts des Signals zu erfassen, der als Zeitpunkt des Auftretens der R-Zacke im QRS-Komplex angesehen wird, wobei der Ausgang des Komparators COMP (4) mit dem Eingang eines Impulsgenerators PULSE GENERATOR (6) verbunden ist, der einen Impuls für die Dauer des Zeitfensters SEARCHING_WINDOW erzeugt, der für die Suche nach der R-Zacke im QRS-Komplex des EKG-Signals verwendet wird, wobei der Ausgang des Impulsgenerators PULSE GENERATOR (6) mit dem Gating-Eingang des Erfassungsmoduls PEAK_DETECTOR (5) und mit dem Eingang des Speichermoduls R_AMPLITUDE_MEMORY (8) zur Speicherung von Informationen über die Amplitude der R-Zacken des EKG-Signals und mit dem Eingang des Speichermoduls R_TIMESTAMP_MEMORY (9) zur Speicherung von Informationen über die Zeitpunkte des Auftretens der R-Zacken des EKG-Signals verbunden ist, wobei der Ausgang des Erfassungsmoduls PEAK_DETECTOR (5) auch mit den Eingängen der Speichermodule R_AMPLITUDE_MEMORY (8) und R_TIMESTAMP_MEMORY (9) verbunden ist, während der Ausgang des Speichermoduls R_AMPLITUDE_MEMORY (8) auch mit dem Eingang des Moduls TH_MODULE (7) verbunden ist, das geeignet ist, einen aktuellen Schwellenwert TH auf der Grundlage der mit dem Erfassungsmodul PEAK_DETECTOR (5) erfassten und mit dem Speichermodul R_AMPLITUDE_MEMORY (8) gespeicherten Amplituden der R-Zacken in den vorhergehenden QRS-Komplexen des EKG-Signals zu bestimmen, wobei der Ausgang des Moduls TH_MODULE (7) auch mit einem der Eingänge des Komparators COMP (4) verbunden ist.

## Revendications

1. Dispositif de détection d'un complexe QRS d'un signal d'électrocardiogramme (ECG) avec une connexion au module de mesure du signal ECG, où le dispositif comprend un module ABS_DIFF_SHORT_MODULE (2) qui détermine le signal représentant la différence entre la valeur instantanée actuelle du signal ECG et une valeur moyenne SHORT_AVG du signal ECG calculée pour un intervalle de temps T_SHORT d'une longueur prédéterminée, et un module ABS_DIFF_LONG_MODULE (3), qui détermine le signal représentant la différence entre la valeur instantanée actuelle du signal ECG et une valeur moyenne LONG_AVG du signal ECG calculée pour l'intervalle de temps T_LONG d'une longueur fixe, dont les entrées sont connectées à une sortie d'un module de mesure ECG_MODULE (1) du signal ECG, tandis que la sortie du module ABS_DIFF_SHORT_MODULE (2) est connectée à l'une des entrées d'un comparateur COMP (4), et que la sortie du module ABS_DIFF_LONG_MODULE (3) est connectée à l'entrée d'un module de détection PEAK_DETECTOR (3), adapté pour détecter pendant une fenêtre temporelle SEARCHING_WINDOW afin de rechercher une onde R dans le complexe QRS, la valeur maximale du signal étant la différence entre la valeur instantanée actuelle du signal ECG et sa valeur moyenne LONG_AVG calculée pour le segment de temps T_LONG, qui est considérée comme l'amplitude de l'onde R dans le complexe QRS, et qui est en même temps adaptée pour détecter le moment de l'apparition de la valeur maximale du signal déterminée de cette manière, ce qui est considéré comme le moment de l'apparition de l'onde R dans le complexe QRS, la sortie du comparateur COMP (4) étant connectée à l'entrée d'un générateur d'impulsions PULSE GENERATOR (6), qui génère l'impulsion avec la durée de la fenêtre temporelle SEARCHING_WINDOW, qui est utilisée pour rechercher l'onde R dans le complexe QRS du signal ECG, tandis que la sortie du générateur d'impulsions PULSE GENERATOR (6) est connectée à l'entrée de déclenchement du module de détection PEAK_DETECTOR (5) et à une entrée du module d'enregistrement de la mémoire R_AMPLITUDE_MEMORY (8) pour stocker des informations sur l'amplitude de l'onde R du signal ECG, ainsi qu'à l'entrée d'un module d'enregistrement de la mémoire R_TIMESTAMP_MEMORY (9) pour stocker des informations sur l'occurrence de l'onde R du signal ECG, tandis que la sortie du module de détection PEAK_DETECTOR (5) est également connectée aux entrées des modules de mémoire R_AMPLITUDE_MEMORY (8) et R_ TIMESTAMP_MEMORY (9), et que la sortie du module de mémoire R_AMPLITUDE_MEMORY (8) est connectée à l'entrée d'un module TH_MODULE (7), adapté pour déterminer une valeur seuil actuelle TH sur la base de l'amplitude de l'onde R détectée par le module de détection PEAK_DETECTOR (5) et stockée dans la mémoire R_AMPLITUDE_MEMORY (8) dans les complexes QRS précédents du signal ECG, la sortie du module TH_MODULE (7) étant également connectée à l'une des entrées du comparateur COMP (4).
